# EUROPEAN PATENT SPECIFICATION

(11) **EP 4 011 360 B1**
(45) Date of publication and mention of the grant of the patent: **13.05.2026**
(21) Application number: 19940905.3
(22) Date of filing: 06.08.2019
(51) Int. Cl.: A61K 8/60, A61K 8/92, A61K 8/86, A61K 8/9789, A61Q 19/00

(54) **COSMETIC COMPLEX AND COMPOSITION FOR MOISTURIZING SKIN AND THEIR USE**
KOSMETISCHER KOMPLEX UND ZUSAMMENSETZUNG ZUR FEUCHTIGKEITSSPENDENDEN PFLEGE DER HAUT UND IHRE VERWENDUNG
COMPLEXE ET COMPOSITION COSMÉTIQUE POUR L'HYDRATATION DE LA PEAU ET LEUR UTILISATION

(43) Date of publication of application: 15.06.2022
(73) Proprietor: Natura Cosméticos S.A., 05106-000 São Paulo SP (BR)
(72) Inventor: DO NASCIMENTO, Selma, 07790-190 Cajamar - SP (BR); ARRUDA COSTA, Andrea, 07790-190 Cajamar - SP (BR); PEREIRA SANDOLIN, Talita, 07790-190 Cajamar - SP (BR); ARANDAS MONTEIRO E SILVA, Silas, 07790-190 Cajamar - SP (BR); DE MIRANDA CHAVES VASQUEZ PINTO, Luciana, 07790-190 Cajamar - SP (BR); BRITO SILVA, Laís, 07790-190 Cajamar - SP (BR); SAVIETTO, Joice, 07790-190 Cajamar - SP (BR); ANDRADE ARCURI, Helen, 07790-190 Cajamar - SP (BR); BELTRAME REIGADA, Juliana, 07790-190 Cajamar - SP (BR); ZIMBARDI, Daniela, 07790-190 Cajamar - SP (BR); DE SOUZA FERREIRA GARCIA, Cinthia Fernanda, 07790-190 Cajamar - SP (BR); GREGÓRIO PINTO, Nicole, 07790-190 Cajamar - SP (BR)
(74) Representative: Simmons & Simmons
(86) International application number: PCT/BR2019/050318
(87) International publication number: WO 2021/022344

(56) References cited:
- EP-A1- 1 600 143
- EP-A1- 3 995 128
- WO-A1-2017/055943
- GB-A- 2 485 483
- US-A- 4 839 164
- US-B2- 8 048 456
- US-B2- 8 815 308
- US-B2- 8 895 082
- US-B2- 8 993 006

## Description

### FIELD OF THE INVENTION

The present invention relates to a cosmetic complex that provides simultaneous skin hydration benefits, as well as a cosmetic composition. Said complex comprises the combination of cocoa butter *(Theobroma cacao),* trehalose and linseed oil *(Linum usitatissimum).* The invention further relates to a moisturizing cosmetic composition (cosmetic product) comprising the complex disclosed herein and a method for moisturizing the skin.

### BACKGROUND OF THE INVENTION

Hydration is essential for a restored skin barrier.

The skin barrier plays a very important role in the body, it comprises cells from the outermost skin layer (horny layer) and substances that bind these cells, such as ceramides, cholesterol, free fatty acids and fats.

As suggested by its own name, it is the body's defense mechanism that acts as a kind of protective layer, preventing external agents from penetrating the skin. Accordingly, this layer retains several harmful substances to which the skin is exposed on a daily basis. When this occurs, in addition to being weakened, the skin has its hydration potential reduced, acquiring a dry appearance and causing a feeling of discomfort and irritation.

Many emollient and humectant ingredients are already known and used in skin hydration compositions. Among them are vegetable oils and butters, such as almond oil, sunflower oil, grape seed oil, linseed oil, castor oil, shea butter, cocoa butter, cupuagu butter, among many others, which in combination with other cosmetic ingredients and even active ingredients form a moisturizing product.

In addition to oils and butters, other ingredients of natural or synthetic origin can also be used in hydration products, including saccharides, disaccharides such as trehalose and polysaccharides.

Trehalose is a non-reducing disaccharide consisting of two glucose units which, together with glycogen, are considered energy reserve substances for yeast.

Each of the ingredients that comprise the complex proposed herein has already been used in skin moisturizing cosmetic compositions. However, said ingredients are always associated with other active ingredients that are not present in the composition of the present invention. Furthermore, nothing has been described so far about the development of a complex capable of offering a mechanism different from those usually known for hydration (occlusion or wetting), the so-called active hydration.

For example, patent US4386067 to Expanscience AS, published in 1983, and GB2485483A, published in 2012, disclose the use of linseed oil or cocoa butter in cosmetic compositions for dry skin.

Likewise, as an example, RO97280 and WO14027163 can be cited, which disclose the use of cocoa butter and trehalose as moisturizing components in skin compositions. Said compositions include ingredients that are not present in the composition of the present invention.

Patent document WO17055943 to BAKEL SRL, despite citing the ingredients in the context of skin care compositions, does not contemplate the possibility of using the components that form the hydration complex of the present invention in combination in a single composition. Furthermore, the compositions claimed herein achieve qualitative constitutions distinct from those described in this prior-art reference.

EP 3995128 concerns a process for obtaining bioactive ingredients, the use of a specific extraction process and new bioactive ingredients from renewable sources, particularly from Amazonia, which can be used in the preparation of cosmetic compositions for treating the skin and hair and/or scalp.

The challenge of obtaining new hydration compositions that provide simultaneous benefits in the treatment of the skin still exists.

So far, no skin hydration composition has been disclosed that comprises the complex formed by cocoa butter *(Theobroma cacao),* trehalose and linseed oil *(Linum usitatissimum),* for use on dry skin, providing the surprising benefit of active hydration, together with other associated benefits.

### BRIEF DESCRIPTION OF THE FIGURES

Figure 1 illustrates the percentage of increase in skin hydration, n = 27 for Group A, wherein the assessment region was the legs.
Figure 2 illustrates the percentage of increase in skin hydration, n = 27 for Group B, wherein the assessment region was the legs.
Figure 3 shows results relative to *S.epidermidis* viability.
Figure 4 shows results relative to *S*. *aureus* viability.

### DESCRIPTION OF THE INVENTION

The present invention is aimed at the technological field of active hydration.

Seeking to overcome the obstacles of the state of the art, the applicant developed a moisturizing composition that provides simultaneous benefits in the treatment of the skin.

In the context of the present invention, simultaneous benefits are understood as cosmetic effects of care, treatment and protection that occur simultaneously and unexpectedly.

These benefits are: skin hydration, improvement of skin elasticity/firmness, reduction of clinical dryness, skin barrier fortification and maintenance of the skin microbiota.

The skin microbiota is maintained in the sense that viability of the most predominant skin bacteria (*S. Epidermidis)*) is favored, while the growth of S. *aureus* is controlled, and the ingredient trehalose provides a prebiotic activity *in vitro.*

Said moisturizing composition comprises a cosmetic hydration complex.

The cosmetic skin hydration complex of the present invention comprises the combination of 0.5% cocoa butter *(Theobroma cacao),* 0.5% trehalose and 0.5% linseed oil *(Linum usitatissimum),* based on the total weight of the final cosmetic composition

The above percentages as well as those indicated in the following examples are based on the total weight of the final cosmetic composition in which the complex will be delivered.

Without limiting the components, in a preferred embodiment, trehalose lipids and total linseed oil are used.

Said complex is capable of providing, in a unique and surprising manner, the benefit of active skin hydration.

By active hydration, in the terms of the present invention, is meant the ability to stimulate the skin to produce its own hydration substances (stimulate endogenous skin mechanisms), that is, molecules that are capable of retaining water in all of the skin layers for a long period of time even if the use of the moisturizing product is stopped.

An stimulation action by endogenous skin mechanisms cannot be predicted. In the present case, the five main endogenous mechanisms that must be accessed and stimulated to ensure effective skin hydration were characterized. As follows:
(1) Stimulation of molecules that retain water in the deeper layers of the skin (dermis) such as hyaluronic acid and other extracellular matrix components (4);
(2) Stimulation of molecules that retain water on the skin surface as natural moisturizing factors, known as NMF (mineral salts);
(3) Stimulation of lipid production and metabolism on the skin surface;
(4) Stimulation of adequate and balanced removal of dead cells from the skin surface (desquamation); and
(5) Stimulation of molecules that carry water and mineral salts to maintain the skin's osmotic balance (such as aquaporins).

It is important to emphasize that via classical mechanisms (occlusion or wetting), the hydration ingredient (or product) needs to be present at all times so that the loss of water to the environment does not occur. On the other hand, it was found that due to the stimulation of endogenous mechanisms, the complex of the present invention surprisingly acts as a "trigger" and the ingredients do not need to be present at all times since from the moment the initial stimulus occurs, production of the skin's own water retaining molecules is promoted. This has been proven by clinical tests that have proven maintenance of hydration for up to 7 days after suspension of use of the product, as described in the tests below.

The present invention has demonstrated that combination of the three active ingredients together has the ability to modulate all endogenous mechanisms in a complementary and unexpected manner.

Said ingredients were expected to act in classic hydration mechanisms such as occlusion and/or wetting and not that the combination thereof could enable active hydration, whose main differential is self-hydration, or maintenance of skin hydration for longer periods of time by stimulating endogenous mechanisms, more specifically five mechanisms.

In another embodiment, the present invention provides cosmetic compositions according to claim 4.

The cosmetically acceptable excipients according to the present invention are those known to the person skilled in the art for making cosmetic bases in various forms, for example, emulsions, creams, gels, serums, and others known to the person skilled in the art.

In yet another embodiment, the present invention provides a method for moisturizing the skin according to claim 6.

Additionally, in the context of the present invention, the moisturizing composition comprises a cosmetic product that contains a body moisturizing formulation.

The body moisturizer is intended for use on dry skin, which uses an active hydration complex preferably made up of cocoa butter, trehalose and linseed oil.

The following examples are representative of embodiments of the invention, without imposing any limitation on its scope.

### EXAMPLES (partly not within the scope of the present invention)

### Example 1. Cosmetic composition according to one embodiment of the present invention.

The following table illustrates one embodiment of a cosmetic composition according to the present invention, which was produced by an usual method known to the person skilled in the art.

**Table 1 - Moisturizing composition**

| Ingredient | (%) | (%) |
|---|---|---|
| | Minimum-maximum | Preferred |
| Dimethiconol | 1-3 | 2 |
| Dimethicone | 0.1-3 | 1 |
| disodium EDTA | 0.1-1 | 0.1 |
| *Elaeis Guinneensis* Oil | 1-3 | 2.5 |
| Perfume | 0.1-0.9 | 0.5 |
| Glycerin | 2-7 | 5 |
| *Hellianthus annuus hybrid* oil | 1-3 | 2 |
| Hydroxyacetophenone | 0.1-1 | 0.3 |
| Lauryl glucoside and polyglyceryl-2 dipolyhydroxystearate | 0.1-1 | 0.5 |
| ***Linum usitatissimum* seed oil** | **0.1-1** | **0.5** |
| ***Theobroma cacao* seed butter** | **0.05-0.9** | **0.1** |
| Olus oil | 1-3 | 2.5 |
| Polyglyceryl-3 caprylate | 0.05-0.9 | 0.1 |
| Pentaerythrityl tetra-di-t-butyl hydroxyhydrocinnamate | 0.01-0.9 | 0.05 |
| Phenoxyethanol | 0.1-0.9 | 0.5 |
| Sodium acrylate copolymer | 0.5-2 | 1.2 |
| Sodium polyacrylate | 0.2-1.2 | 0.8 |
| Tocopherol acetate | 0.05-0.9 | 0.1 |
| **Trehalose** | **0.5-3** | **1.2** |
| *Zea Mays* Corn Starch | 0.1-2 | 1 |
| Water | q.s.p. | q.s.p. |

Thereafter tests were carried out in order to demonstrate the simultaneous benefits of the invention.

### Example 2. Demonstration of active hydration.

The compositions according to example 1 were tested to assess their hydration effects.

The study was designed and carried out with 54 participants who had apparent skin dryness, where the level of dryness was scored as follows:

| Classification | Definition |
|---|---|
| 0 | No dryness: Nourished, smooth skin with no evidence of dryness |
| 1 | Slight dryness: slightly evident lines (cracks), texture slightly irregular to the touch - absence of flaking and off-white appearance. |
| 2 | Moderate dryness: evident and not very intense lines (cracks), slightly irregular texture to the touch - flaking and slight off-white appearance |
| 3 | Mild dryness: evident and moderately intense lines (cracks), moderately irregular texture to the touch - flaking and slightly off-white appearance |
| 4 | Moderately intense dryness: evident and very intense lines (cracks), very irregular texture to the touch - flaking and intense off-white appearance |
| 5 | Severe dryness: evident and severely intense lines (cracks), severely irregular texture to the touch - flaking and very intense off-white appearance. |

At the time of inclusion in the study, the participants were instructed to discontinue the use of any topical product 7 days prior to the start of the study. On the first day of the study, a clinical and instrumental assessment measured the initial cutaneous status of each of the participants. At this time the participants were divided into two groups as follows:
- Group A - Low/Moderate dryness intensity; and
- Group B - Moderate/High intensity of dryness.

Then, the research participants were instructed to apply the product of the invention on their legs skin in the manner they are used to when applying a moisturizing cream.

The methodology consisted of the objective analysis performed by obtaining data by evaluating photographic images and by short kinetic evaluation (at the beginning of the study T0, after 15 min, after 4 hours, after 8 hours, after 24 hours, after 48 hours), and by long kinetic evaluation (after 28 days and after 7 days of suspension of use of the investigational product).

Analysis of the images obtained made it possible to assess the increased skin hydration even after discontinuing the use of the product under investigation.

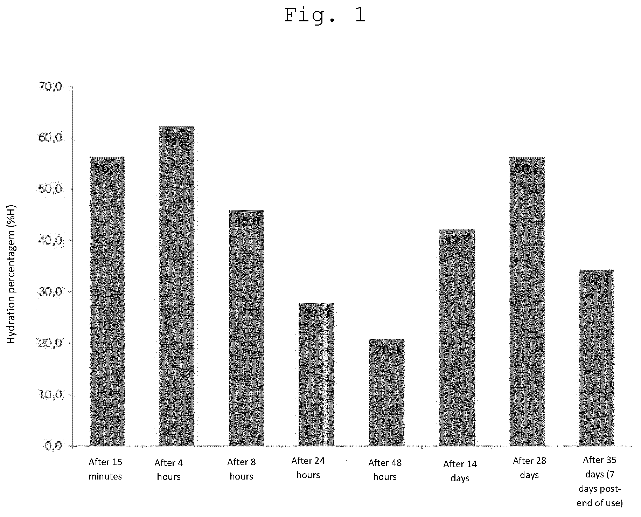

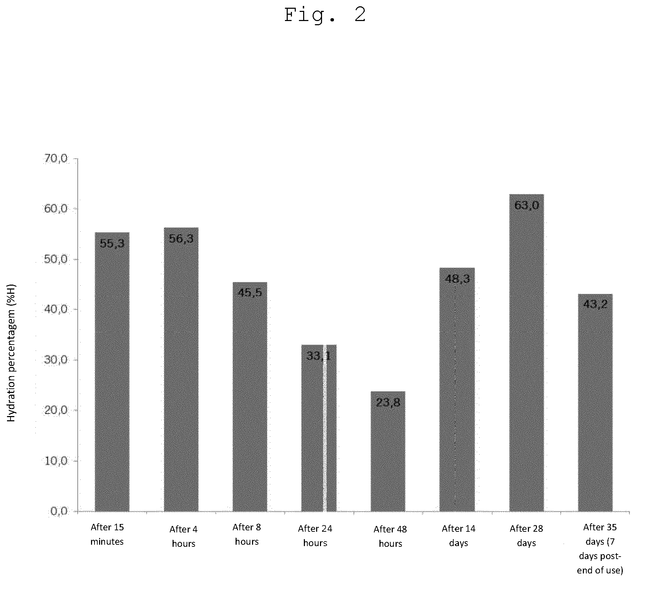

The graphics in figures 1 and 2 show the obtained results in terms of percentage increase in hydration after application of the investigational product. Use of the investigational product was shown to confer positive results in terms of skin hydration in the short and long terms. The main results observed are as follows:
- The investigational product provided a significant increase in leg skin hydration after 15 minutes, 4, 8, 24 and 48 hours of application;
- For research participants with light to moderate dryness and desquamation of the skin of the legs, the investigational product increased the hydration level of the skin of the legs by up to 62.3% after application;
- For research participants with moderate to severe dryness and desquamation of leg skin, the investigational product increased leg skin hydration level by up to 63% after application;
- the product significantly increased leg skin hydration 7 days after discontinuation of home use compared to the baseline condition and the control, indicating maintenance of skin hydration for up to 7 days after discontinuation of use; and
- 100% of the research participants have shown improvement in leg skin hydration after treatment with the investigational product.

There were no reports or evidence of adverse reactions during the study.

### Example 3. Elasticity and Firmness Test.

The same research participants of example 2 were subjected to tests to assess the benefits of skin elasticity and firmness.

Advantageous results were obtained, demonstrating that the product improved skin firmness and elasticity after 14 and 28 days of use:
- Up to 4.7% increase in skin firmness after 14 days of use (81% of participants showed increased skin firmness after 14 days);
- Up to 3.2% increase in skin elasticity after 14 days of use (78% of participants showed increased elasticity after 14 days);
- Up to 9.9 % increase in skin firmness after 28 days of use (100% of participants showed increased skin firmness after 28 days);
- Up to 7.7% increase in skin elasticity after 28 days of use (96% of participants showed increased elasticity after 28 days).

A comparative analysis with another formulation was able to demonstrate the benefits of the composition of the present invention. The results can be seen in table 2 below.

**Table 2 - Elasticity and Firmness Results**

| **Test** | **Formula of the Invention** | **Natura Everyday Cherry and Hazelnut Formula** | **Natura Everyday Cotton (Dry Skin) Formula** |
|---|---|---|---|
| Firmness (28 days) | + 9.9% | + 3.9% | Not performed |
| % of Participants with increased Firmness | 100% | 77.3% | Not performed |
| Elasticity (28 days) | + 7.7% | + 4.6% | Not performed |
| % of Participants with increased Firmness | 96% | 81.8% | Not performed |
| Hydration | + 23.8% (48 hours) | + 9.0% * (30 hours) | + 5.0% (24 hours) |
| % of Participants with increased hydration | 96% (48 hours) | 70% * (30 hours) | 95% (24 hours) |
| Barrier Fortification (28 days) | + 11.9% | + 8.0% | + 40.0% |
| % of participants with an increase (28 days) | 100% | 76.9% | - |

### Example 4. Barrier Fortification Test

This test aimed to assess the potential of the composition of the invention to strengthen the skin barrier responsible for protecting and ensuring hydration.

The same research participants of example 2 were subjected to tests to assess barrier fortification.

From the results it was concluded that the product of the invention was able to provide a strengthened skin barrier after 14 and 28 days of use of the composition. The results were as follows:
- Up to 6.9% increase in skin barrier after 14 days of use (89% of participants showed increased skin barrier); and
- Up to 11.9% increase in skin barrier after 28 days of use (100% of participants showed increased skin barrier).

### Example 4 - Protein expression

Complex 1 of the preferred embodiment according to the present invention was assessed (complex 1: 1.2% trehalose + 0.1% cocoa butter + 0.5% linseed oil). Protein expression results demonstrating activation in five natural hydration mechanisms are shown below:

**Table 3 - Protein Expression Results**

| | |
|---|---|
| Protein confirmation | 1.2% Trehalose, 0.5% linseed and 0.1% Cocoa Butter |
| P-value<0.005 | |
| Hyaluronic acid | 20.7% |
| Filaggrin / NMF Processing | 106.3%/56.4% |
| Lipid Synthesis | 31.0% |
| Aquaporin | 66.2% |
| Desquamation | 52.9%/16.7% |

### Example 5 - Microbiota

The same research participants of example 2 were subjected to tests to assess the results on the skin microbiota.

A comparative test was carried out to assess the impact of the formulation of the invention on the skin microbiota, particularly on the maintenance of *S*. *aureus and S*. *epidermidis* species.

For that end, a cell viability assay was carried out, which consisted of:
- Incubating the remaining fragments and removing them one at a time: t10min, t30min, t60min;
- Removing a VITRO-SKIN fragment and placing it in a conical tube (15mL) containing 10mL of sterile deionized water and vortexing it for 30 seconds;
- Repeating the procedure for the other replicas;
- Performing serial dilutions (10 ⁻⁴ and 10 ⁻³), plating and incubating. Dilutions were plated in the differential medium for *S*. *aureus and S*. *epidermidis* (MSA or BP).

Then, a recovery test was performed, which consisted of:
- Incubating the replicas (n=3) of the VITRO-SKIN fragments for 60 minutes;
- Removing a VITRO-SKIN fragment and incubating it in 10 mL TBS medium at 37°C;
- Remove 0.2mL of medium containing VITRO-SKIN at 30, 60 and 120 minutes and proceed to plating.
- Performing serial dilutions (10 ⁻⁴ and 10 ⁻³), plating and incubating. Dilutions were plated in the differential medium for *S*. *aureus and S*. *epidermidis* (MSA or BP).

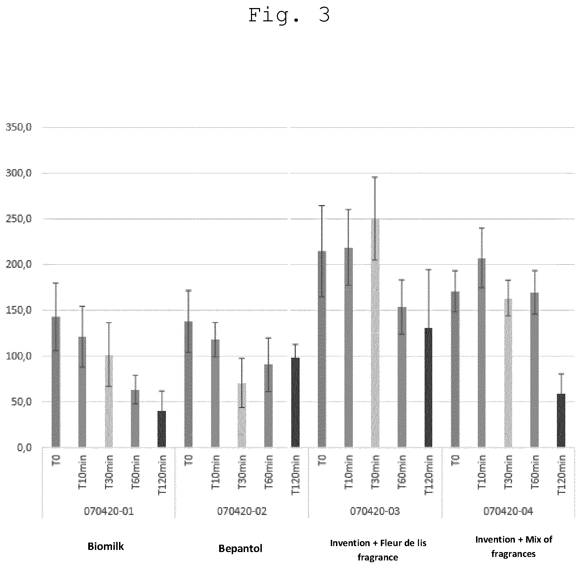

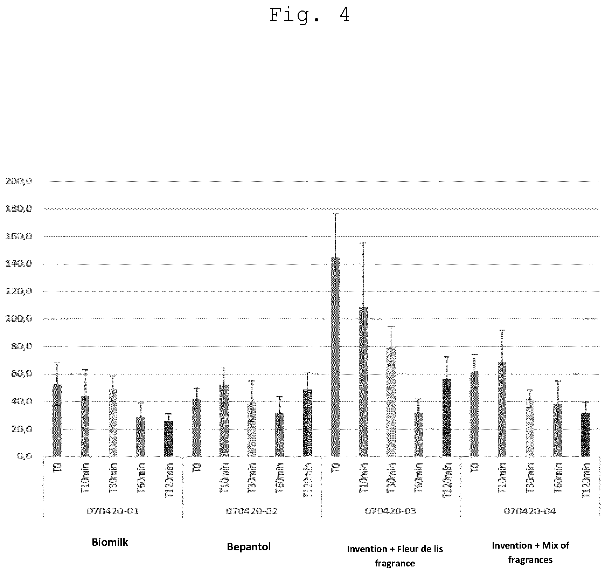

The results are shown in Figures 3 and 4. The tested samples are:
01- Biomilk (natural humectant)
02- Bepantol (Dexpanthenol)
03- Formula of the Invention + Fleur de Lis fragrance
04- Formula of the Invention + mix of fragrances.

According to the graph illustrated in Figure 3, the following results were found relative to the viability of *S.epidermidis*:
- Biomilk is the product with the greatest impact on the *S*. *epidermidis* viability, progressively reducing the count and reaching 1/3 of the initial count after 2h of contact with the product;
- Bepantol provides an initial drop of 20% in S. *epidermidis* viability and was maintained until the end of the trial;
- The formulation of the invention + Fleur de Lis fragrance did not affect S. *epidermidis* viability during the first 30 minutes of testing. After 1 hour, there was a 10% reduction and this value remained stable until the end of the test;
- The formulation of the invention + mix of fragrances showed no change in S. *epidermidis* viability within the first hour of the test, showing that no components used in the fragrances has a negative impact on the most predominant bacteria of the microbiota.

According to the graph illustrated in Figure 4, the following results were found relative to the viability of *S*. *aureus*:
- The formulation of the invention + Fleur de Lis fragrance had the least impact on *S*. *aureus* viability immediately after application;
- The formulation of the invention + mix of fragrances reduced the *S*. *aureus* viability by 50% after application;
- After 60 minutes of exposure to the formulation, all products evaluated had the same impact on *S*. *aureus* viability.

It was noted that the tested product provided the maintenance of the skin flora, as the Gram-, Yeast/Mold species and Gram+ species showed fluctuations over time with use of the product.

The descriptive findings of the microbiota found in this study remain within favorable conditions to conceive maintenance of healthy skin characteristics.

The presence of fragrances to be used in the body moisturizer variants did not impact the viability of the studied bacteria. Therefore, the results apply to all fragrances.

According to these tests, the benefits of "balance preservation" and "balance maintenance" of the skin microbiota can be used.

### Example 6 - Barrier formation

The state of the art correlates a balanced microbiota with a fortified skin barrier.

The test was carried out under the following conditions:
- 490 samples;
   - 40% pre-sequencing loss;
- 13% unidentified bacteria.

### Clinical efficacy panel:

- 54 volunteers (41 ± 13 years). Phototypes II, III and IV;
- Wash out of topical products for 3 days prior to the start of the study;
- Samples were acclimated for 30 minutes under a controlled environment (20 ± 2°C and 50 ± 5% RH);
- Dry skin (self-reported or by clinical evaluation);
- Tested product: complex 1 according to the preferred embodiment of the present invention;
- Mode of use: Applying daily at least once a day, except in areas marked with tape (contralateral control);
- Microbiota collection performed in the forearm, legs (both sides) and neck regions;
- Clinical efficacy data collected from the forearms and legs.

The results are shown in the following table:

**Table 4: Summarized data from the statistical analysis of the significance of changes in the skin barrier. P values.**

| Comparison groups | Group A | | Group B | |
|---|---|---|---|---|
| | Control | Investigational Product | Control | Investigational Product |
| **Δ*E _{D0} vs.* Δ*E _{D14}*** | 0.2159 (not significant) | <0.0001 (significant) | 0.3886 (not significant) | 0.0133 (significant) |
| **Δ*E _{D0} vs.* Δ*E _{D28}*** | 0.1520 (not significant) | <0.0001 (significant) | 0.9192 (not significant) | <0.0001 (significant) |

## Claims

1. A cosmetic composition **characterized in that** it comprises a COSMETIC SKIN HYDRATION COMPLEX, **characterized by** comprising the combination of 0.5% cocoa butter *(Theobroma cacao),* 0.5% trehalose and 0.5% linseed oil *(Linum usitatissimum),* based on the total weight of the final cosmetic composition

2. NON-THERAPEUTIC USE of the cosmetic composition as defined in claim 1, for skin hydration.

3. The NON-THERAPEUTIC USE, according to claim 2, for maintenance of the skin microbiota.

4. A COSMETIC COMPOSITION, as defined in claim 1, together with cosmetically acceptable excipients.

5. The COMPOSITION, according to claim 4, **characterized in that** it is a body moisturizer.

6. A METHOD FOR MOISTURIZING THE SKIN, **characterized in that** it comprises applying to dry skin an effective amount of the cosmetic composition as defined in claim 1, or the cosmetic composition as defined in claim 4.

## Patentansprüche

1. Kosmetische Zusammensetzung, **dadurch gekennzeichnet, dass** sie einen KOSMETISCHEN HAUTFEUCHTIGKEITSKOMPLEX umfasst, **gekennzeichnet durch** das Umfassen der Kombination von 0,5% Kakaobutter (Theobroma cacao), 0,5% Trehalose und 0,5% Leinsamenöl (Linum usitatissimum), basierend auf dem Gesamtgewicht der endgültigen kosmetischen Zusammensetzung.

2. NICHTTHERAPEUTISCHE VERWENDUNG der in Anspruch 1 definierten kosmetischen Zusammensetzung zur feuchtigkeitsspendenden Pflege der Haut.

3. NICHTTHERAPEUTISCHE VERWENDUNG nach Anspruch 2 zur Aufrechterhaltung der Hautmikrobiota.

4. KOSMETISCHE ZUSAMMENSETZUNG wie in Anspruch 1 definiert, zusammen mit kosmetisch verträglichen Hilfsstoffen.

5. ZUSAMMENSETZUNG nach Anspruch 4, **dadurch gekennzeichnet, dass** sie eine Körperfeuchtigkeitspflege ist.

6. VERFAHREN ZUR FEUCHTIGKEITSSPENDENDEN PFLEGE DER HAUT, **dadurch gekennzeichnet, dass** es das Auftragen einer wirksamen Menge der in Anspruch 1 definierten kosmetischen Zusammensetzung oder der in Anspruch 4 definierten kosmetischen Zusammensetzung auf trockene Haut umfasst.

## Revendications

1. Composition cosmétique **caractérisée en ce qu'**elle comprend un COMPLEXE COSMÉTIQUE POUR L'HYDRATATION DE LA PEAU, **caractérisé en ce qu'**il comprend la combinaison de 0,5 % de beurre de cacao (Theobroma cacao), de 0,5 % de tréhalose et de 0,5 % d'huile de lin (Linum usitatissimum), sur la base du poids total de la composition cosmétique finale.

2. UTILISATION NON THÉRAPEUTIQUE de la composition cosmétique telle que définie dans la revendication 1, pour l'hydratation de la peau.

3. UTILISATION NON THÉRAPEUTIQUE selon la revendication 2, pour le maintien du microbiote cutané.

4. COMPOSITION COSMÉTIQUE telle que définie dans la revendication 1, conjointement avec des excipients cosmétiquement acceptables.

5. COMPOSITION selon la revendication 4, **caractérisée en ce qu'**elle est un hydratant pour le corps.

6. PROCÉDÉ D'HYDRATATION DE LA PEAU **caractérisé en ce qu'**il comprend l'application sur une peau sèche d'une quantité efficace de la composition cosmétique telle que définie dans la revendication 1, ou de la composition cosmétique telle que définie dans la revendication 4.
